# EUROPEAN PATENT APPLICATION

(11) **EP 4 008 316 A1**
(43) Date of publication of application: **08.06.2022**
(21) Application number: 21211890.5
(22) Date of filing: 02.12.2021
(51) Int. Cl.: A61K 9/20, A61K 9/28

(54) **A FILM COATED TABLET FORMULATION COMPRISING DAPAGLIFLOZIN AND METFORMIN HYDROCHLORIDE**

(30) Priority: 03.12.2020 TR 202019589
(71) Applicant: SANOVEL ILAC SANAYI VE TICARET A.S., Istanbul 34460 (TR)
(72) Inventor: PEHLIVAN AKALIN, Nur, 34460 Istanbul (TR); SÜNEL, Fatih, 34460 Istanbul (TR); OZDEN, Aydan, 34460 Istanbul (TR); ARSIN, Gülcan, 34460 Istanbul (TR)
(74) Representative: Sevinç, Erkan

(57) **Abstract**

The present invention relates to a film coated tablet formulation comprising dapagliflozin and metformin hydrochloride wherein dapagliflozine has a d (0.9) particle size between 20 µm to 140 µm. Further, the present invention also relates to a simple, rapid, cost effective, time-saving and industrially convenient process.

## Description

### Field of the Invention

The present invention relates to a film coated tablet formulation comprising dapagliflozin and metformin hydrochloride wherein dapagliflozine has a d (0.9) particle size between 20 µm to 140 µm. Further, the present invention also relates to a simple, rapid, cost effective, timesaving and industrially convenient process.

### Background of the Invention

Diabetes mellitus is a group of disorders of carbohydrate metabolism in which the action of insulin is diminished or absent through altered secretion, decreased insulin activity or a combination of both factors. There are two main types of diabetes; Type 1 and Type 2:
Type 1 diabetes occurs because the insulin-producing cells of the pancreas (beta cells) are damaged. In Type 1 diabetes, the pancreas makes little or no insulin, so sugar cannot get into the body's cells for use as energy. People with Type 1 diabetes must use insulin injections to control their blood glucose.

In Type 2 diabetes, the pancreas makes insulin, but it either doesn't produce enough, or the insulin does not work properly. This diabetes occurs most often in people who are over 40 years old and overweight and type 2 diabetes is the most common type, affecting more than 171 million people worldwide.Type 2 diabetes may sometimes be controlled with a combination of diet, weight management, and exercise. However, treatment also may include oral glucose-lowering medications or insulin injections.

Dapagliflozin is a sodium-glucose cotransporter 2 inhibitor (SGLT2). SGLT2 is a carrier responsible for the reabsorption of most of the glucose from the lumen of the renal tubule. SGLT2 is expressed in proximal renal tubules. By inhibiting SGLT2, dapagliflozin reduces the reabsorption of the filtered glucose and lowers the renal threshold for glucose. This improves urinary glucose excretion and blood glucose control. Dapagliflozin, also known as (1S)-1,5-Anhydro-1-C-[4-chloro-3-[(4-ethoxyphenyl)methyl]phenyl]-D-glucitol or (2S,3R,4R,5S,6R)-2-(3-(4-etoxybenzyl)-4-chlorophenyl)-6-hydroxymethyltetrahydro-2H-pyran-3,4,5-triol is represented by the structure of Formula I.

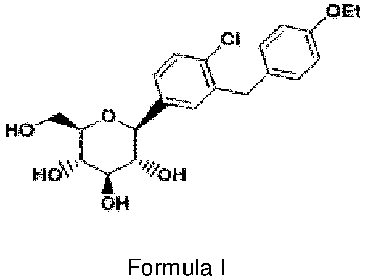

Dapagliflozin was first disclosed in patent US 6515117 (2003, Bristol-Myers Squibb).

Metformin is antidiabetics having an orally-administrated biguanide structure. Metformin hydrochloride is a white to off-white crystalline compound and it is freely soluble in water and practically insoluble in acetone, ether and chloroform. Oral doses of metformin are generally recommended in the range of 500 to 2500 mg a day and a single dose may vary from 250 to 1000mg. It is used singly or in combination with sulfonylureas, alpha-glucosidase inhibitors, or insulin.

The chemical name of metformin hydrochloride is 1,1-dimethylbiguanide hydrochloride, has the following chemical structure of Formula II.

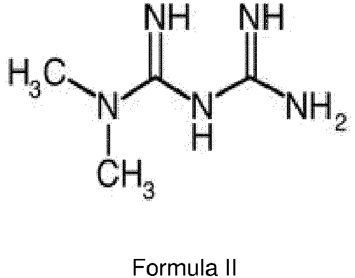

Metformin was first disclosed in patent US 3174901 (1965, Jan Marcel Didier Aron-Samuel).

The two oral hypoglycemic agents dapagliflozin and metformin hydrochloride belong to the group of sodium-glucose symporter-2 (SGLT-2) inhibitors and biguanides, respectively, and the two have different hypoglycemic mechanisms. Synergistic effects can also reduce adverse reactions.

Dapagliflozin and metformin, sold under the brand name Xigduo XR among others, is a fixed-dose combination anti-diabetic medication used as an adjunct to diet and exercise to improve glycemic control in adults with type 2 diabetes.

EP2498759 (A2) discloses an immediate release pharmaceutical formulation which includes a tablet or capsule formulation comprising metformin and the dapagliflozin or its propylene glycol hydrate.

Although several formulations comprising dapagliflozin and metformin HCI are known in the art, finding a good or even the optimal form with regard to stability and dissolution profile remains a considerable challenge.

The dapagliflozin is hygroscopic in nature. It absorbs moisture and forms sticky lumps which are difficult to process and handle, and which may ultimately lead to content uniformity issues in the dosage form. The low solubility and stability of dapagliflozin base as compared to its solvates may lead to poor bioavailability of the drug. Also, as known, metformin is a very poorly compressible active substance and metformin presents in high amounts in a composition. The problem causes some content uniformity problems.

There is thus still a need for film coated tablet formulation comprising dapagliflozin and metformin hydrochloride that provides a tablet having the desired stability, dissolution profile, hardness and compressibility, in another words the disadvantages seen in the active substances will able to overcome. The formulation has been developed by using standard techniques which is simple and cost-effective method.

### Detailed Description of the Invention

Another object of the present invention is to provide a film coated tablet formulation comprising dapagliflozin and metformin hydrochloride having the desired stability, dissolution profile, hardness and compressibility.

The main object of the present invention is to eliminate problems caused by active substances and bringing additional advantages to the relevant prior art.

In this invention, combining more than one molecule in one dosage form increases the patient's compliance. However, while this combination is increasing the patients' quality of life, combining more than one molecule in one dosage form also reduces side effects.

As used here in, 'particle size' means the cumulative volume size distrubition as tested by any conventionally accepted method such as the laser diffraction method (i.e. malvern analysis). The term d (0.1) means, the size at which 10% by volume of the particles are finer and d (0.5) means the size at which 50% by volume of the particles are finer and d (0.9) means the size at which %90 by volume of the particles are finer.

We have found that dapagliflozin having the following particle sizes is very important for formulation. Especially, it positively affects the dissolution properties and powder homogenization. The obtained tablets have the desired dissolution profile. The powder is more homogeneous. The content uniformity of the tablets obtained from the more homogeneous powder is more ideal. Therefore, it provides better bioavailability.

According to an embodiment of the present invention, a film coated tablet formulation comprising dapagliflozin and metformin wherein dapagliflozine has a d (0.9) particle size between 20 µm to 140 µm.

According to an embodiment of the present invention, dapagliflozine has a d (0.9) particle size between 22 µm to 130 µm, between 25 µm to 120 µm, between 30 µm to 110 µm, between 35 µm to 100 µm, between 40 µm to 98 µm, between 45 µm to 95 µm, between 47 µm to 90 µm, between 50 µm to 85 µm.

According to an embodiment of the present invention, dapagliflozine has a d (0.1) particle size between 3 µm to 60 µm.

According to an embodiment of the present invention, dapagliflozine has a d (0.1) particle size between 5 µm to 55 µm, between 6 µm to 50 µm, between 7 µm to 45 µm, between 8 µm to 40 µm, between 9 µm to 30 µm.

According to an embodiment of the present invention, dapagliflozine has a d (0.5) particle size between 8 µm to 90 µm.

According to an embodiment of the present invention, dapagliflozine has a d (0.5) particle size between 9 µm to 85 µm, between 10 µm to 80 µm, between 11 µm to 75 µm, between 11 µm to 70 µm, between 15 µm to 60 µm, between 18 µm to 55 µm, between 20 µm to 50 µm, between 25 µm to 45 µm.

Another problem encountered while developing formulations is the flowability-problem and compressibility of metformin HCI, which makes the production difficult. It has been observed that this problem is overcome by using the described below particle sizes of metformin HCI.

According to an embodiment of the present invention, metformin HCI has a d (0.1) particle size between 2 µm to 60 µm.

According to an embodiment of the present invention, metformin HCI has a d (0.1) particle size between 4 µm to 55 µm, between 5 µm to 50 µm, between 6 µm to 45 µm, between 6 µm to 40 µm, between 6 µm to 30 µm.

According to an embodiment of the present invention, metformin HCI has a d (0.5) particle size between 8 µm to 90 µm.

According to an embodiment of the present invention, metformin HCI has a d (0.5) particle size between 9 µm to 85 µm, between 10 µm to 80 µm, between 11 µm to 75 µm, between 11 µm to 70 µm, between 15 µm to 60 µm, between 18 µm to 55 µm, between 20 µm to 50 µm, between 22 µm to 45 µm.

According to an embodiment of the present invention, metformin HCI has a d (0.9) particle size between 75 µm to 200 µm.

According to an embodiment of the present invention, metformin HCI has a d (0.9) particle size between 80 µm to 190 µm, between 82 µm to 180 µm, between 83 µm to 175 µm, between 85 µm to 170 µm, between 88 µm to 168 µm, between 92 µm to 163 µm, between 100 µm to 158 µm, between 110 µm to 155 µm.

According to an embodiment of the present invention, the amount of dapagliflozin is 0.05% to 5.0% by weight, preferably 0.1% to 3.0% by weight in the total composition.

According to an embodiment of the present invention, the amount of metformin HCI is 55.0% to 80.0% by weight, preferably 59.0% to 75.0%, more preferably 63.0% to 70.0% by weight in the total composition.

In general terms, excipients provided in a formulation may positively or negatively influence the physicochemical and pharmacokinetic properties, e.g. the solubility, absorption, bioavailability of an active agent. For this reason, the excipients which accompany an active agent have to be selected in a careful and conscious manner while a formulation is developed. The formulations should have no physicochemical incompatibility between the active agents and the excipients.

According to an embodiment of the present invention, the film coated tablet formulation comprise pharmaceutically acceptable excipients selected from fillers, binders, disintegrants, lubricants or mixtures thereof.

Suitable fillers are selected from the group comprising microcrystalline cellulose, lactose, starch, mannitol, calcium hydrogen phosphate dihydrate, dicalcium hydrogen phosphate anhydrate, calcium phosphate trihydrate, silicon dioxide, neutral pellets, magnesium carbonate, magnesium oxide, maltodextrin, maltose, medium chain triglycerides or mixtures thereof.

According to an embodiment of the present invention, the filler is microcrystalline cellulose.

According to an embodiment of the present invention, the amount of fillers is 5.0% to 30% by weight, preferably 7.0% to 25.0%, more preferably 10.0% to 20.0% by weight in the total composition.

The advantages of the present invention are even more significant, as the problem of homogeneity is even more likely to occur when two active substances are incorporated in one final dosage form, especially when two actives is used very different regarding the amount. Improved content uniformity efficiently contributes to a marked increase in bioavailability. Improved content uniformity also favors to avoid toxicity in the otherwise possible event that the amount of drug substance would be too high.

Also, as known, metformin is a very poorly compressible active substance and in the present invention, metformin presents in high amounts in the tablet. It was surprisingly found that when prepared the tablet formulation with at least one suitable binder, it was observed that the desired tablet hardness, content uniformity and compressibility, the desired friability.

Suitable binders are selected from the group comprising polyvinylpyrrolidone (povidone), hydroxypropyl methylcellulose, lactose anhydrous, pregelatinized starch, calcium carbonate, calcium phosphate dibasic, calcium phosphate tribasic, calcium sulphate, hydroxypropylcellulose, carboxymethylcellulose, methyl cellulose, ethyl cellulose or mixtures thereof.

According to an embodiment of the present invention, the binder is polyvinylpyrrolidone.

According to one embodiment of the invention, at least one binder is dissolved in a solvent and a binder solution is obtained. For this reason, it ensures that active agents and excipients adhere to each other in an ideal way and provides homogeneous granule units. In this way, possible blend uniformity and content uniformity problems that may be seen in the product are prevented.

According to an embodiment of the present invention, the amount of binders is 2.0% to 15% by weight, preferably 2.0% to 10.0%, more preferably 3.0% to 8.0% by weight in the total composition.

Dapagliflozin is used small proportion that can lead to considerable problems during the manufacture of the composition with regard to the uniformity of the content of active agent in the individual composition units. Because of problems uniformity of the content, the active substance may interact with several excipients. It reflects that content uniformity play important role in the dissolution of the drug. Furthermore, using the right disintegrant helps to ensure uniformity of the content. That is, the disintegrant plays an important role for the dissolution profile.

Suitable disintegrants are selected from the group comprising croscarmellose sodium, sodium starch glycollate, crospovidone, sodium alginate, gums, starch and magnesium aluminum silicate or a mixture thereof.

According to an embodiment of the present invention, the disintegrant is croscarmellose sodium.

According to an embodiment of the present invention, the amount of disintegrants is 1.0% to 10.0% by weight, preferably 1.0% to 8.0%, more preferably 1.0% to 6.0% by weight in the total composition.

Suitable lubricants are selected from the group comprising magnesium stearate, silica colloidal anhydrous, sodium stearyl fumarate, talc, polyethylene glycol, stearic acid, aluminum silicate or mixtures thereof.

According to an embodiment of the present invention, the lubricant is magnesium stearate.

According to an embodiment of the present invention, the amount of lubricants is 0.1% to 8.0% by weight, preferably 0.1 to 5%, more preferably 0.1 to 3% by weight in the total composition.

Film coating is important for the present invention, because dapagliflozin absorbs moisture, film coating protect the composition against the moisture and light to maintain the stability.

According to an embodiment of the present invention, the film coated tablet comprises coating agents for film coating.

Suitable coating agents are selected from the group comprising polymethacrylates, hydroxypropyl methylcellulose, lactose monohydrate, talc, hydroxypropyl cellulose, polyvinyl alcohol (PVA), polyethylene glycol (PEG), talc, glycerine, polyvinyl alcohol-polyethylene glycol copolymers (Kollicoat^{®} IR), ethylcellulose dispersions (Surelease^{®}), polyvinylprolidone, polyvinylprolidone-vinyl acetate copolymer (PVP-VA), Iron oxide all kinds of Opadry^{®}, pigments, dyes, titanium dioxide, coloring agent or mixtures thereof.

According to an embodiment of the present invention, the coating agents are polyvinyl alcohol, talc, titanium dioxide, polyethylene glycol, iron oxide.

According to an embodiment of the present invention, the amount of coating agents is 2.0% to 10.0% by weight, preferably 3.0 to 8.0% by weight in the total composition.

According to an embodiment of the present invention, the film coated tablet of the present invention comprises dapagliflozin and metformin HCI, polyvinylpyrrolidone, microcrystalline cellulose, croscarmellose sodium, magnesium stearate.

The tablet of the present invention may be prepared, using standard techniques and manufacturing processes well known in the art, such as direct compression, wet granulation or dry granulation, hot melt granulation, hot melt extrusion, fluidized bed granulation, extrusion/spheronization, slugging, spray drying and solvent evaporation.

According to an embodiment of the present invention, the film coated tablet is prepared wet granulation. So, an easy method was created to eliminate the disadvantages of both active ingredients.

### Example 1:

| **Active ingredient and excipient** | **Ingredients in one tablet (%) (by weight)** |
|---|---|
| Dapagliflozin | 0.05 - 5.0 |
| Metformin HCI | 55.0 - 80.0 |
| Polyvinylpyrrolidone | 2.0 - 15.0 |
| Microcrystalline cellulose | 5.0 - 30.0 |
| Croscarmellose sodium | 1.0 - 10.0 |
| Magnesium stearate | 0.1 -8.0 |
| Film coating agents | 2.0 - 10.0 |
| **Total** | **100** |

### Example 2:

| **Active ingredient and excipient** | **Ingredients in one tablet (%) (by weight)** |
|---|---|
| Dapagliflozin | 0.3 |
| Metformin HCI | 68.5 |
| Polyvinylpyrrolidone | 5.5 |
| Microcrystalline cellulose | 16.8 |
| Croscarmellose sodium | 2.4 |
| Magnesium stearate | 1.0 |
| Film coating agents | 5.5 |
| **Total** | **100** |

### A process for example 1 or 2;

a) Sieving dapagliflozin, metformin HCI, microcrystalline cellulose and croscarmellose sodium and then mixing,
b) Dissolving polyvinylpyrrolidone in a solvent, preferably pure water and obtained binder solution.
c) Spraying the binder solution to the mixture at step (a),
d) Drying,
e) Sieving the granules,
f) Adding magnesium stearate and then mixing,
g) Compressing the mixture to form of tablet,
h) Coating tablets with film coating agents.

## Claims

1. A film coated tablet formulation comprising dapagliflozin and metformin wherein dapagliflozine has a d (0.9) particle size between 20 µm to 140 µm.

2. The film coated tablet formulation according to claim 1, wherein, dapagliflozine has a d (0.1) particle size between 3 µm to 60 µm.

3. The film coated tablet formulation according to claim 1, wherein, dapagliflozine has a d (0.5) particle size between 8 µm to 90 µm.

4. The film coated tablet formulation according to claim 1, wherein metformin HCI has a d (0.1) particle size between 2 µm to 60 µm.

5. The film coated tablet formulation according to claim 1, wherein metformin HCI has a d (0.5) particle size between 8 µm to 90 µm.

6. The film coated tablet formulation according to claim 1, wherein metformin HCI has a d (0.9) particle size between 75 µm to 200 µm.

7. The film coated tablet formulation according to claim 1, wherein the amount of dapagliflozin is 0.05% to 5.0% by weight, preferably 0.1% to 3.0% by weight in the total composition.

8. The film coated tablet formulation according to claim 1, wherein the amount of metformin HCI is 55.0% to 80.0% by weight, preferably 59.0% to 75.0%, more preferably 63.0% to 70.0% by weight in the total composition.

9. The film coated tablet formulation according to claim 1, wherein the film coated tablet formulation comprise pharmaceutically acceptable excipients selected from fillers, binders, disintegrants, lubricants or mixtures thereof.

10. The film coated tablet formulation according to claim 9, wherein binders are selected from the group comprising polyvinylpyrrolidone (povidone), hydroxypropyl methylcellulose, lactose anhydrous, pregelatinized starch, calcium carbonate, calcium phosphate dibasic, calcium phosphate tribasic, calcium sulphate, hydroxypropylcellulose, carboxymethylcellulose, methyl cellulose, ethyl cellulose or mixtures thereof.

11. The film coated tablet formulation according to claim 10, wherein the binder is polyvinylpyrrolidone.

12. The film coated tablet formulation according to claim 10, wherein the amount of binders is 2.0% to 15% by weight, preferably 2.0% to 10.0%, more preferably 3.0% to 8.0% by weight in the total composition.

13. The film coated tablet formulation according to claim 9, wherein disintegrants are selected from the group comprising croscarmellose sodium, sodium starch glycollate, crospovidone, sodium alginate, gums, starch and magnesium aluminum silicate or a mixture thereof.

14. The film coated tablet formulation according to claim 13, wherein the disintegrant is croscarmellose sodium.

15. The film coated tablet formulation according to claim 13, wherein the amount of disintegrants is 1.0% to 10.0% by weight, preferably 1.0% to 8.0%, more preferably 1.0% to 6.0% by weight in the total composition.
